# EUROPEAN PATENT APPLICATION

(11) **EP 1 898 211 A1**
(43) Date of publication of application: **12.03.2008**
(21) Application number: 06018835.6
(22) Date of filing: 08.09.2006
(51) Int. Cl.: G01N 27/414, G01N 27/12

(54) **Insulated substrate impedance transducer**

(71) Applicant: Université Catholique De Louvain, 1348 Louvain-La-Neuve (BE); Facultés Universitaires Notre-Dame de la Paix, 5000 Namur (BE)
(72) Inventor: Pampin, Rémi Sébastien, 92400 Courbevoie (FR); Flandre, Denis, B-1170 Bruxelles (BE); Moreno-Hagelsieb, Luis, B-1348 Louvain-la-Neuve (BE); Foultier, Boris, B-1450 Chastre (BE); Remacle, José, B-5020 Malonne (BE)
(74) Representative: Bird, William Edward

(57) **Abstract**

Electronic transducer (10) and a method for detecting and/or characterising target materials or physicochemical stimuli in an external medium (8) using the electronic transducer (10). The electronic transducer (10) comprises a sensing element (3) featuring a variable conductance when exposed to a stimulus from the external medium and a first and second electrode (5a,5b) spaced apart on or in a sensing material surface of a substrate, the sensing element being provided in or on the substrate and being located between the first and the second electrodes (5a,5b) forming a pair of sensing electrodes for sensing a change in conductance of the sensing element (3) in a direction substantially parallel to the sensing material surface, at least one of the sensing electrodes (5a,5b) being electrically insulated from the sensing element (3) by a dielectric layer (4), so as to be capacitively coupled to the sensing element (3). An insulating layer or target specific layer (7) may optionally be provided covering the sensing element (3) and optionally the sensing electrodes and being adapted for contact with the external medium (8). Electrical measurements made between the pair of sensing electrodes (5a,5b) are influenced by the impedance of the sensing element formed as a channel (3) which is affected by the presence of the medium (8) to be tested.

## Description

### Technical field of the invention

The present invention relates to electronic transducers, more particularly to insulated substrate impedance transducers or ISITs, and to a method for detecting the presence of and/or characterising targets materials appearing in solid, liquid or gaseous media by electronic transduction of physico-chemical stimuli through impedance measurements. The electronic transducers according to the present invention can, for example, be used for biochemical analysis, for electro-physical characterisation of materials with a particular work function, Fermi level or redox potential, or for electronic transduction of electric, magnetic or mechanical inputs.

### Background of the invention

Research and development activities for measurement systems-on-a-chip currently imply typical structures of electrical devices associated to specific measurement techniques. In this context, a distinction has to be made between intrinsic parameters of a device, modified by design and fabrication, and extrinsic properties of the device due to the external reactive medium which is in contact with a surface of the system, the medium including target materials and stimulation sources.

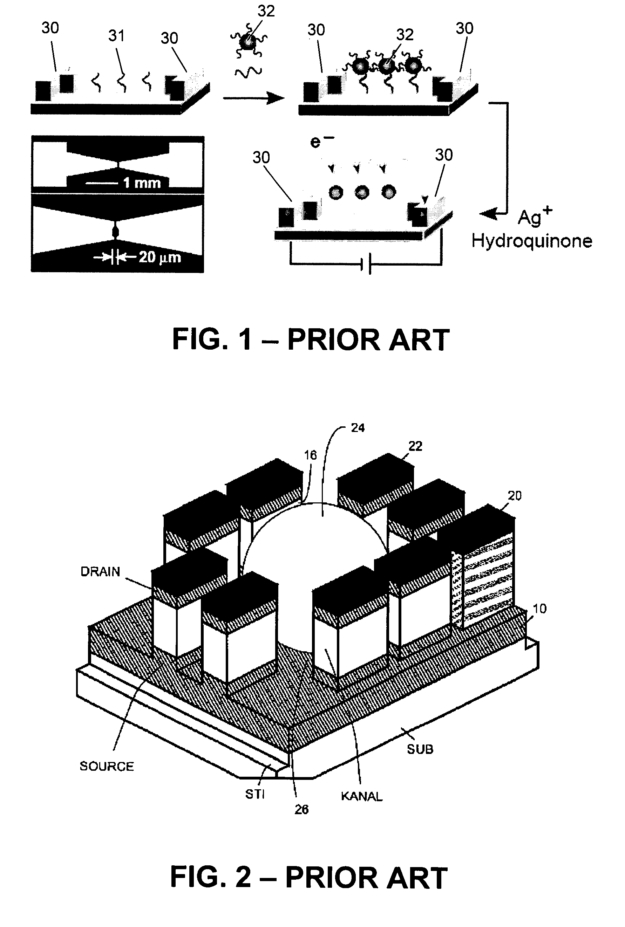

In a first category, passive electrodes access extrinsic properties of the reactive medium such as DC resistance, AC capacitance or AC complex impedance. Basic electrochemical cells, for example, use macroscopic "work" and "reference" electrodes, preferably defined as respectively "drive" and "sense" electrodes when integrated on a chip [C. Guiducci et al., Biosens. Bioelectron. 19 (2004), p.781-787)]. Fig. 1 illustrates metal electrodes 30 for conductance measurements, which is an example of this first category. Binding sites 31 are provided between the electrodes 30, for selectively binding target sample 32 provided with gold labels. After binding of the target sample 32, silver (Ag) is precipitated by hydroquinone around the gold labels. The electrical resistivity of the Ag precipitates (i.e. the real component of its impedance) is measured by applying a DC current between the two electrodes 30 and measuring the resulting voltage difference.

In another, second category, smart transducers with built-in sensitive elements rely on their intrinsic parameters such as AC/DC transconductance or impedance, which vary as an image of outward conditions. Fig. 2 illustrates field-effect smart transducers for cell monitoring, which is an example of this second category as illustrated in DE-102 54 158. Vertical transistors comprising source 26, channel 16 and drains 22 are arranged on an epitaxial layer 10 on a substrate SUB in a ring. A sample 24 such as a biological cell alters the operation of the vertical transistors, e.g. their AC/DC transconductance is changed. The conduction state of sensitive channel element 16, depends on the ionic concentration, i.e. pH, of the reactive medium in the neighbourhood of living cell 24, following its electrochemical activity in physiological solution . Differentiated drain contacts 20 allow sensor multiplexing with regard to a common source contact 10, in order to get a spatial resolution of the cell's activity.

Another category of devices has an intermediate design and lies in-between the above-described approaches without clearly combining them. Examples of such intermediate designs are e.g. Electrolyte-Insulator-Semiconductor or EIS structures [A. Abdelghani et al., Materials Science and Engineering C26, p.542-545 (2006)] and photoconductive or chemically sensitive resistors.

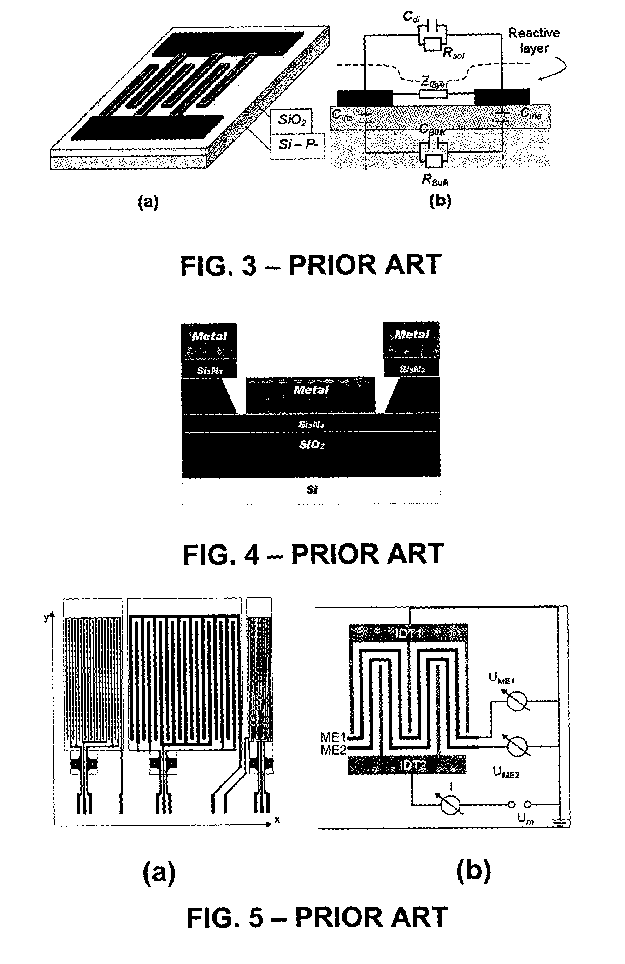

Regarding the first category, innovations generally apply to electrode geometries, such as described for example in M. Hollis et al., WO 93/22678 for micropatterns, P. Van Gerwen et al., WO 97/21094 for sidewalls, U. Schlecht et al., Analytica Chimica Acta, in press (2006) for nanogaps, M. Gheorghe et al., Biosens. Bioelectron. 19, p.95-102 (2003) for surface materials (noble metals), M. Yi et al., Biosens. Bioelectron. 20, p.1320-1326 (2005) for microelectronic compatible metals, C. Escher et al., DE 100 49 902 A1 for interface dielectrics, P.-G. Su et al., Sens. Actuators 113, p. 837-842 (2006) for resistive measurement methods, L. Moreno et al., Sens. Actuators B98, p. 269-274 (200) for capacitive measurement methods, V. F. Lvovich et al., Sens. Actuators B, in press (2006) for impedimetric measurement methods, or T.G. Drummond et al., Nat. Biotechnol. 21 (10), p.1192-1199 (2003) for electrochemical measurement methods. In these cases, the quality of sensing interfaces is particularly critical since they have to endure external reactions possibly enhanced by measurement current flows. Most popular chip designs comprise interdigitated array-based electrodes known as IDE (interdigitated electrodes) or IDA (interdigitated arrays), offering a relative simple way of fabrication. Fig. 3(a), 3(b), 4 and 5 illustrate some common shapes known for IDE. Fig. 3(a) and (b) respectively show a top view of an IDE and the corresponding interdigit electrical model. Fig. 4 shows 2-level electrodes with self-aligned fingers. Another possibility is a planar IDE, which is well suited for smart sensing approaches such as, for example, impedance spectroscopy [J.-G. Guan et al., J. Bioscience and Bioengineering 97(4), p.219-226 (2004)] or multi-layer complex dielectrometry. This is illustrated in Fig. 5(a) and (b). Fig. 5(a) shows a device comprising multiple spatial wavelengths, i.e. different dimensions of and distances between sets of interdigitated electrodes, which device is suitable for being used in dielectrometry and Fig. 5(b) shows a 4-points measurement system. Both systems illustrated in Fig. 5 can be used for IDE multiplexing.

According to the dielectrometry method [R. Igreja et al., Sens. Actuators A 112, p.291-301 (2004)], digits of various spatial periodicity permit to sense different material depths, and a subsequent reconstruction algorithm allows discriminating the contribution of each layer in the overall impedance. It is worth noting that mechanical supports lie at a similar distance from measurement electrodes than reactive surface layers. Since the properties of the supports do not respond to extrinsic stimuli, they introduce parasitic elements in the electrical model used for data interpretation, which subsequently affects measurement resolution.In the world of smart transducers (second category), semiconductor-based pioneers lead to hundreds of applications since their early developments in the 1970's [P. Bergveld, Sens. Actuators B88 (2003), p.1-20]. Such sensors, also called field-effect sensors, which are built around metal oxide semiconductor or MOS transistors, require fully DC-compatible electrical accesses to the reactive medium and the semiconductor body. The devices therefore feature highly doped semiconductor regions called "source" and "drain" that connect a lightly doped "channel", which may be of opposite polarity, i.e. doping type. Detection occurs in the sensitive channel element, whose conduction state depends on the electric charges and workfunctions of surface reactive materials [D.T. Van Anh et al., IEEE Sensors Journal 4(3) (2004)]. Field-effect transistors have been used as chemical sensors through modification of the gate insulator or gate electrode geometry, involving various surface layers [X.-L. Luo et al., Sens. Actuators B97, p.249-255 (2004)] and advanced processed supports such as e.g. cantilevers [E.B. Cooper et al., Appl. Phys. Lett. 79, 23 (2001), p.3875-3877] or nanowires [J.-I. Hahn et al., Nano Lett. 4, 1, p.51-54 (2004)].

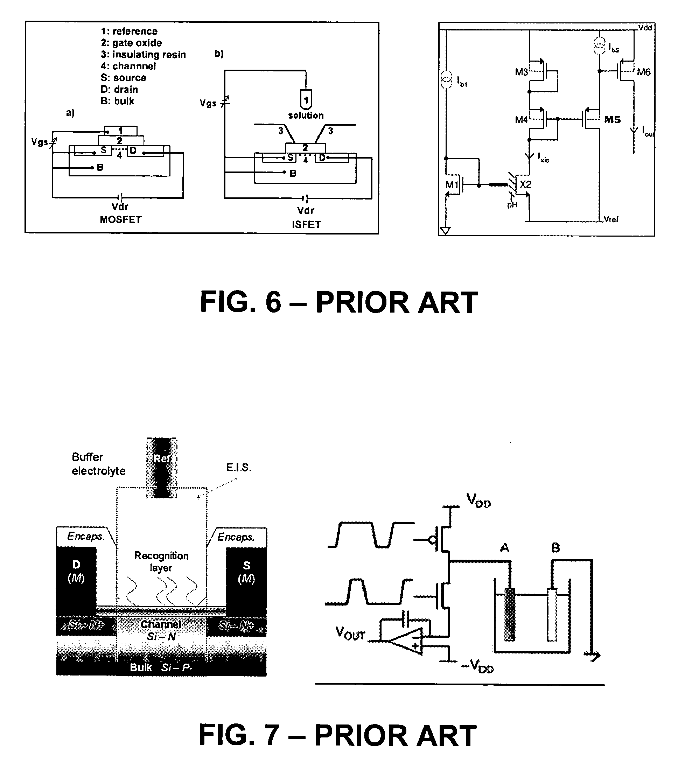

A CHEMFET (chemically sensitive field-effect transistor) is a type of field effect transistor acting as a chemical sensor. It is a structural analog of a MOSFET transistor, where the charge on the gate electrode is applied by a chemical process. It can be used to detect atoms, molecules, and ions in liquids and gases. An ISFET (ion-sensitive field-effect transistor) is the best known sub-type of CHEMFET devices. In an electrolyte, the channel conduction of an ISFET is modulated by electro-active species adsorbed on the gate area, around a bias potential defined with an immersed reference electrode. In this way, the ISFET is used to detect ions in electrolytes. Alternatively, an ENFET (enzyme field effect transistor) is a CHEMFET specialised for detection of specific enzymes. Field-effect transducers may be compared with Kelvin probes since they react to electrostatic and thermodynamic extrinsic properties. Intrinsic transfer characteristics of such sensors are usually measured in DC (static current gain monitoring between source and drain) or AC (dynamic analysis of surface capacitive couplings between gate and other terminals) modes. Fig. 6 illustrates, at the left hand side, a metal semiconductor field-effect transistor (MOSFET) and an ion-sensitive field-effect transistor (ISFET), which can, for example, be used for pH sensing. The right hand side of Fig. 6 illustrates a DC transconductance measurement system. Fig. 2 is another example of ISFET arrangement.

EIS (Electrolyte Insulator Semiconductor) cells can be seen as an extension of MOS capacitances where an electrolyte replaces the top metal access. Fig. 7 illustrates some electrochemical setups. At the left hand side of Fig. 7 the relationship between an ISFET and an EIS is illustrated. A transistor is formed with a horizontal channel region formed between a drain (D) and a source (S). Above the channel region a recognition layer is provided, e.g. it allows binding of a target to the layer. This binding affects the operation of the transistor which can be measured by application of signals to and/or reading of signals from the drain and source. Considering the EIS part only (dashed), impedance measurements are done between a reference electrode Ref and bulk silicon Bulk Si-P- through an electrolyte, giving access to the complex surface capacitance of the device which may be changed in the presence of target material, and to the vertical impedance of the channel which may also be affected by field-effect due to the presence of target material. At the right hand side of Fig. 7 a capacitance measurement system is illustrated. Capacitive measurements are performed between a reference electrode B, which is in electrolytical contact with a reactive medium, and a work electrode A which is made of a semiconductor body covered with a thin insulator. Complex capacitance parameters measured include the extrinsic impedance contribution of surface reactive materials as well as the intrinsic impedance of the semiconductor/insulator interface. In other words, EIS structures combine the measurement of both extrinsic medium properties and intrinsic device parameters, possibly modulated by a field-effect perpendicular to the top insulated surface.

Alternatively, semiconducting resistors can be compared to the channel part of a transistor, excluding source and drain regions, on which a couple of DC contacts are performed. The lateral ohmic resistance of the film is thereby directly measured e.g. as a function of incident illumination [S.U. Son et al., Sens. Actuators A11 (2004), p.100-106] or field-effect, occurring on the semiconductor base.

In recent times, a growing attention has been paid to new sensitive materials such as polymers, composites and nanomaterials, for use in sensors for detecting, for example, humidity [P.-G. Su et al., Sens. Actuators 113, p.837-842 (2006)], gas [G. Hagen et al., Sens. Actuators B, in press 2006], IR and UV light [J. D. Hwang et al., Thin Solid Films 491, p.276-279 (2005)] or magnetic field [J. Schotter et al., Biosens. Bioelectron. 19 p.1149-1156 (2004)]. Despite they react through physical and chemical interaction instead of a field-effect, the transduction principle of such devices is a conductance change of the film between DC-compatible accesses. It has to be noted that the distinction previously made between extrinsic properties and intrinsic parameters of the device vanishes in such case since the built-in sensing element chemically reacts with the outward medium.

The critical point of the latter transducers is the contact quality at the electrode/film interface, which has not been fully envisioned at that time. Recently, work has been published in this field which notices an effect due to the interface capacitance of a composite film on metallic electrodes [G. Hagen et al., Sens. Actuators B, in press 2006]. However, the effect is not completely characterized and is attributed to a modification of the chemical reaction itself.

During the last decade, a mature technology has been developed to detect silver-enhanced gold nanoparticles in a biochemical application, which has been described in EP 1 376 111 for detecting hybridised DNA strands labelled with silver-enhanced gold nanoparticles. It consists of an aluminium micro-IDE which is patterned, positioned on top of a 400nm-thick silicon dioxide and covered with a 100nm-thick alumina layer. A base wet oxide is thermally grown on a standard silicon substrate (10¹⁵ P-doped), whereas a top interface layer is obtained after anodizing pure deposited aluminium.

A good efficiency of such structures was demonstrated in capacitive detection of high silver densities, following the coupling introduced by 0.5 µm to 1 µm conductive labels between insulated 1 µm to 2 µm-spaced electrode fingers. However, the sensor's efficiency for a silver coverage lower than 30 % can only be preserved at the cost of reducing their dimensions below 1 µm, especially reducing digit spacing. At best, the technique currently achieves electronic detection of lower concentrations of hybridized DNA down to 0.05 nM, but leading to a capacitance increase of a few tenth of pico-Farad only (about 0.5 pF for 1 nM of DNA, corresponding to an increase of 40 % with respect to the base value).

### Summary of the invention

It is an object of the present invention to provide an alternative or good apparatus and method for detecting the presence of and/or characterising target materials in solid, liquid or gaseous media by electronic transduction of physico-chemical stimuli through impedance measurements.

The above objective is accomplished by a method and device according to the present invention.

In a first aspect of the present invention, a solid-state electronic transducer is provided for testing an external medium. The electronic transducer comprises: a sensing element made of sensing material featuring a variable conductance when exposed to a stimulus from the external medium, and a first and a second electrode spaced apart on or in a sensing material surface of a substrate, the sensing element being provided in or on the substrate and being located between the first and the second electrodes, the first and the second electrodes forming a pair of sensing electrodes allowing to sense a change in conductance of the sensing element in a direction substantially parallel to the sensing material surface, wherein at least one of the first and second electrodes is electrically insulated from the sensing element by a dielectric layer, so as to be capacitively coupled to the sensing element.

The solid-state electronic transducer preferably has the first and second electrodes such that a measurement signal applied to these electrodes for sensing a change in conductance of the sensing element does not change or modulate the conductance of the sensing element. For sensing, a signal may be applied to the first electrode, and the measurement result may be read at the second electrode. Alternatively, a signal may be applied to and the measurement result be read at the first electrode, the second electrode being kept at constant potential, e.g. being grounded.

Advantages of the solid-state electronic transducer according to embodiments of the present invention are:
- it does not need ohmic or drain and source accesses,
- it provides both spatial and temporal information related to distributed conduction states and subsequent dielectric relaxation constants throughout the sensing element, e.g. channel,
- it is easy to manufacture, and
- it allows low-cost impedance measurements.

The pair of sensing electrodes may both be electrically insulated from the sensing element by the dielectric layer.

It is a further advantage of the transducer according to embodiments of the present invention that insulated electrodes are electrically coupled with semiconductor elements, which allows benefiting from dielectric impedance changes induced by field-effect as well as chemical reaction, illumination or mechanical constraint.

The transducer may optionally comprise a target specific insulating layer, also called application specific layer, covering the sensing element and/or the sensing electrodes and insulating these from the external medium, the insulating layer or application specific layer being adapted to contact with the external medium. The insulating layer may be adapted to contact with the external medium for providing the stimulus. The insulating or application specific layer may be a non-conductive layer. Such non-conductive layer may be formed by passivation, oxidation, nitridation or by depositing an insulating substance such as a paint of lacquer of similar insulating coating. In other words, according to embodiments of the present invention, the top surface of the sensing electrodes may be covered by an isolation or application specific layer. For example, the top surface of the sensing electrodes may be covered by a layer which selectively binds specific target materials in order to increase the sensitivity of the transducer for these specific target materials.

According to embodiments of the invention, the sensing element may be formed on or in a bulk mechanical support. The sensing material surface preferably faces away from the support. The solid-state electronic transducer may furthermore comprise a third access terminal forming a back-gate electrode at a side of the bulk mechanical support different from, e.g. away from or opposite to the side where the sensing element is provided.

An advantage hereof is that the back-gate electrode allows tuning of the working range of the solid-state electronic.

The pair of sensing electrodes may be formed of a conductive or semiconducting material. For example, the sense electrodes may be formed of aluminium fingers.

An oxidised doped semiconductor substrate, e.g. silicon substrate, preferably thinly oxidised, i.e. the oxidation layer having a thickness below 100 nm, may form a bulk mechanical support, the sensing element and the dielectric layer.

The sensing material may be a semiconductor material.

The sensing material may a doped semiconductor material.

According to embodiments of the invention, the sensing material may form a channel in the substrate.

The dielectric layer may comprise one insulator material, a combination of insulator materials, one semiconductor material having dielectric properties, a combination of semiconductor materials having dielectric properties, or a combination of at least one insulator material and at least one semiconductor material having dielectric properties.

The insulator material may, for example, be one of an oxide, a nitride or a polymer.

The transducer may have a top surface, and the top surface of the transducer may be provided with conductive, e.g. metallic, or semiconductive particles or grains. The surface coverage with such conductive or semiconductive particles or grains may be at least between 0% and 30% but may preferably be as high as possible, e.g. may reach substantially 100%. These conductive, e.g. metallic, or semiconductive particles or grains may have the function of labels and may also be provided for selectively binding specific target materials. The presence of conductive, e.g. metallic, or semiconductive particles or grains on the transducers surface leads to a decrease of capacitance cut-off frequency.

In a further aspect, the invention provides a method for detecting the presence of and/or characterising target materials or physico-chemical stimuli in an external medium. The method comprises:
- measuring a first impedance of a sensing element of an electronic transducer, the electronic transducer furthermore comprising a first and second access terminal forming a pair of sensing electrodes for measuring the conductance of the sensing element, at least one of the sensing electrodes being electrically insulated from the sensing element by a dielectric layer so as to be capacitively coupled to the sensing element,
- providing the medium comprising the target material to a measurement surface of the transducer,
- measuring a second impedance of the sensing element after provision of the medium to the electronic transducer, and
- from the difference between the first and second impedances of the sensing element determining the presence of and/or characterising the target material.

The measuring may be performed by complex impedance spectroscopy, in which case the pair of sensing electrodes are used for applying an ac signal and measuring the ac response. The complex impedance depends on the parallel conductivity and the capacitance(s) introduced by the insulated electrode(s). The transducer is adapted so that ac or transient signals applied to the electrodes do not modulate the conductance of the sensing element.

The target material may be a conductive compound, an insulating material, a semi-conducting material, a biochemical species, an ionised atom or molecule, a charged particle appearing in solid, liquid or gaseous media.

The physico-chemical stimulus may be light, temperature, pressure, flow, mass, a magnetic stimulus, a mechanical stimulus or an electrical stimulus.

It is an advantage of embodiments according to the present invention that insulated electrodes are electrically coupled with semiconductor elements, which allows benefiting from smart dielectric impedance changes induced by field-effect as well as chemical reaction, illumination or mechanical constraint.

It is a further advantage of embodiments according to the present invention that it presents a new design approach, which is associated to a powerful frequency- and time-domain measurement method, which is known in theoretical literature but which is not familiar with integrated sensors.

Particular and preferred aspects of the invention are set out in the accompanying independent and dependent claims. Features from the dependent claims may be combined with features of the independent claims and with features of other dependent claims as appropriate and not merely as explicitly set out in the claims.

Although there has been constant improvement, change and evolution of devices in this field, the present concepts are believed to represent substantial new and novel improvements, including departures from prior practices, resulting in the provision of more efficient, stable and reliable devices of this nature.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

Fig. 1 illustrates a device comprising metal electrodes for conductance measurements according to the prior art.
Fig. 2 illustrates smart transducers for field-effect cell monitoring according to the prior art.
Fig. 3 illustrates a surface IDE (Fig. 3(a)) and a corresponding interdigit electrical model (Fig. 3(b)) according to the prior art.
Fig. 4 illustrates a device comprising 2-level electrodes with auto-aligned fingers according to the prior art.
Fig. 5 illustrates a device comprising multiple spatial wavelengths between digits, the device being suitable for being used in dielectrometry (Fig. 5(a)) and a 4-point measurement structure (Fig. 5(b)) according to the prior art.
Fig. 6 illustrates a MOSFET and an ISFET (left) and a DC transconductance measurement circuit (right) according to the prior art.
Fig. 7 illustrates the relationship between an ISFET and an EIS (left) and a capacitance measurement system (right) according to the prior art.
Fig. 8a and 8b show cross-sections of transducer devices according to embodiments of the present invention.
Fig. 9 shows a cross-section of a device according to embodiments of the present invention and a corresponding electrical model.
Fig. 10 shows a top view of the surface of a prototype of a transducer according to embodiments of the present invention (left) and a corresponding distribution model (right).
Fig. 11 illustrates a lumped element electrical model with 3 poles (left) and a reconstructed relaxation spectrum with 3 lobes (right) according to the prior art.
Fig. 12A and Fig. 12B show simulations of measurements performed with field-effect sensors according to embodiments of the present invention for a blank surface (dashed line) and for a surface covered with 25% silver grains area coverage (full line).
Fig. 13A and Fig. 13B illustrate admittance changes of field-effect sensor prototypes with 1 nM of 534-mer DNA targets according to embodiments of the present invention before a biochemical process (dashed line) and for a 30% silver grains area coverage (full line).

In the different figures, the same reference signs refer to the same or analogous elements.

### Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Moreover, the terms top, bottom and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

The invention will now be described by a detailed description of several embodiments of the invention. It is clear that other embodiments of the invention can be configured according to the knowledge of persons skilled in the art without departing from the true spirit or technical teaching of the invention, the invention being limited only by the terms of the appended claims.

The present invention relates to electronic transducers and more particularly to insulated substrate impedance transducers or ISITs and to a method for detecting the presence of and/or characterising target materials appearing in solid, liquid or gaseous media by electronic transduction of physico-chemical stimuli through impedance measurements. An electronic transducer accepts an input or stimulus which can be non-electrical and provides an associated electrical output. It is thus a device that converts energy from one form into another of an electrical nature, such as voltage, current, or resistance for example. Almost all electronic transducers require additional circuits to produce the electrical output signal, e.g. voltage or current, that represents the initial quantity of the input or stimulus. The electronic transducer according to embodiments of the present invention operates by having a physical property that is altered by an external stimulus or input. Each input from the real world produces a unique output of the transducer.

The electronic transducer or ISIT and method according to the present invention can be used with electromagnetic or physico-chemical inputs such as voltage, light, mechanical constraint, pressure, or temperature. It can, for example, furthermore be used for biochemical analysis or for electro-physical characterisation of materials with an electric charge, a particular workfunction, Fermi level or redox potential, or for electronic transduction of electric, magnetic or mechanical inputs. Target materials that can be detected by the transducer and method according to the invention may be any target material that comprises conductive, insulating or semi-conducting compounds, biochemical species, ionised atoms or molecules, charged particles. For example, the device and method according to the present invention can be used for analysis of DNA, viruses or others in view of the detection of pathogens in medical, agro-nutritional, security fields, ....

An objective of the present invention is to couple insulated surface electrodes strongly with sensing elements or channels (see further), in order to benefit from smart dielectric impedance changes induced by a suitable stimulus, such as for example the field-effect, a chemical reaction, illumination or mechanical constraint.

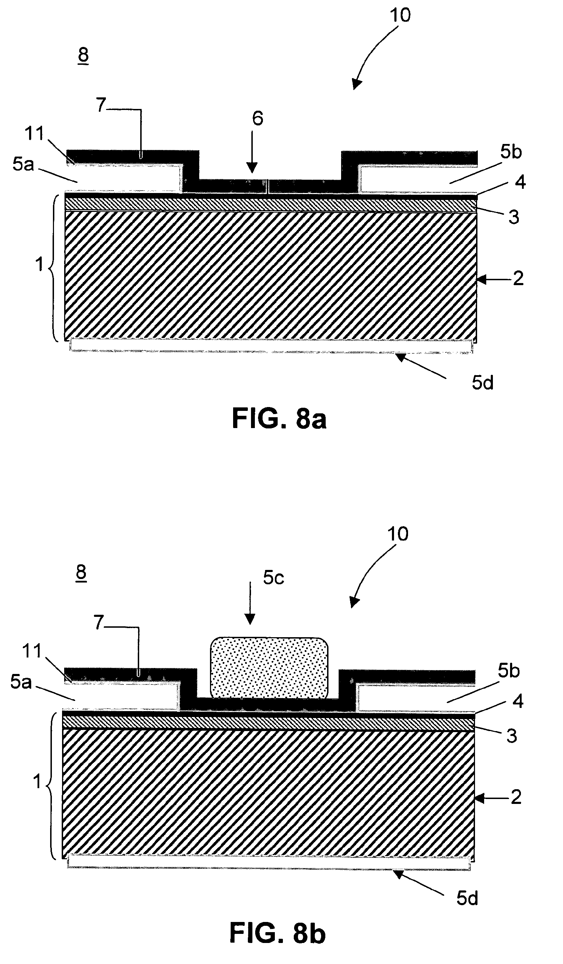

Fig. 8a and 8b illustrate examples of an integrated electronic transducer or ISIT 10 according to embodiments of the present invention. The electronic transducer or ISIT 10 comprises a substrate 1. The substrate 1 comprises a bulk mechanical support 2, e.g. a semiconductor substrate. Preferably, the bulk mechanical support 2 may comprise silicon. However, any other suitable semiconductor material, such as, for example, III-V semiconductor compounds (e.g. GaAs), may also be used. According to other embodiments of the invention, the bulk mechanical support 2 may comprise another material than a semiconductor material. For example, the bulk mechanical support may also comprise an insulating material such as silicon on glass or any other suitable insulating material. According to still other embodiments, the bulk mechanical support may also comprise a polymer material, such as polycarbonate, polystyrene, polypropylene or any other suitable polymer material.

On top of the bulk mechanical support 2 the substrate 1 comprises a layer of sensing material featuring a variable conductance when exposed to a stimulus, e.g. a channel 3, and a dielectric layer 4.

The layer of sensing material, e.g. channel 3, may be formed by a layer which can be, but does not necessarily have to be, of the same material as the bulk mechanical support 2. The layer of sensing material, e.g. channel 3, may be formed of a semiconductor material, such as e.g. silicon or a III-V semiconductor compound, which may be pure or may be doped by introduction of defects or impurities such as e.g. boron, phosphorous or arsenic, or can be any other material in which a conductance change as a response to a stimulus can be exploited. In case the layer of sensing material, e.g. channel 3, is formed of a semiconductor material, local or differential doping of the layer of sensing material, e.g. channel 3, below the electrodes or below the reactive region (see further) may be exploited to optimize the device properties. The material of which the layer of sensing material, e.g. channel 3, is formed, may also be called the sensing material. Accordingly, the channel 3 itself may also be called the sensing element of the transducer or ISIT 10. According to the present invention, any sensing material able to provide a conductance variation in response to a stimulus, for example in response to the field-effect, can be used as built-in sensing element or channel 3 in the ISIT 10 provided that a suitable dielectric layer 4 is intercalated between the material and at least one electrode (see further).

The dielectric layer 4 can comprise any suitable dielectric material provided that is designed so as to match impedance requirements at working frequencies, depending on the conductance range of the sensing material.

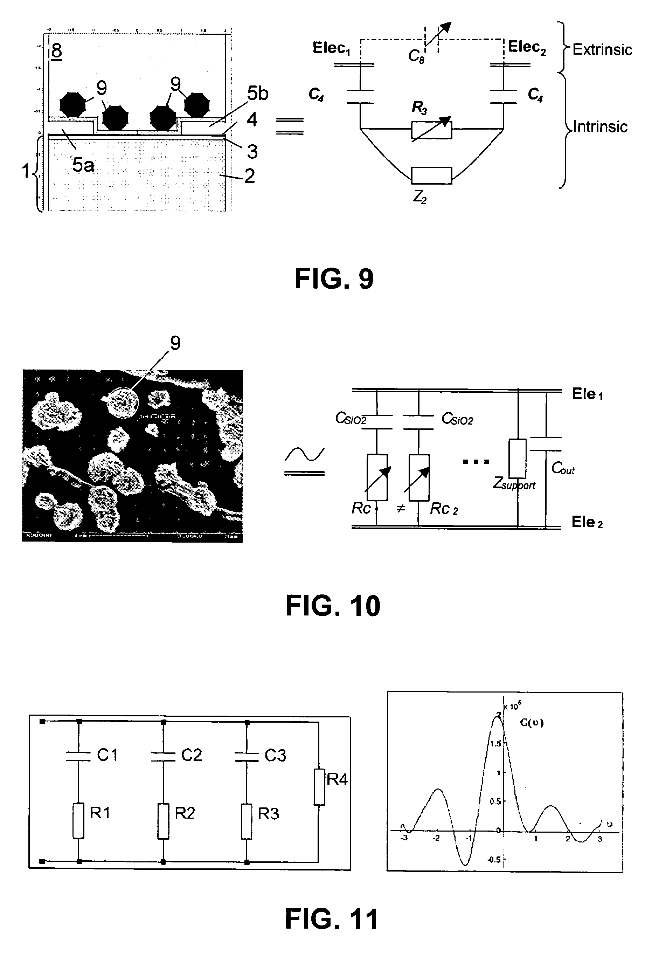

On the basis of the electrical model shown in Fig. 9 (right side) for example, approximating Z₂ by a pure capacitance C₂, the global impedance Z_{eq} measured between Elec₁ and Elec₂ depends on the frequency:
- at a characteristic frequency given by f_{c} ~ 1/(6.28* R₃* C₂), Z_{eq} ≅ {R₃ in series with C₄};
- at lower frequencies Z_{eq} ≅ C₄;
- at higher frequencies Z_{eq} ≅ C₂;
where Cᵢ depends on the dielectric permittivity εᵢ and Rᵢ depends on the conductance σᵢ of associated materials, Rᵢ and Cᵢ also being functions of the device geometry (i.e. electrode surface and shape factor, thickness of the layers forming the electrodes, ...).

The dielectric layer 4 may, for example, comprise one single insulator material or a combination of insulators such as e.g. oxides, nitrides or polymers, as well as one or a combination of semiconductor materials having dielectric properties. The dielectric layer 4 may be a single layer or a stack of layers. The dielectric layer 4 forms an insulation between the layer of sensing material, e.g. channel 3, of the transducer or ISIT 10 and at least one of its electrodes (see further).

According to an embodiment of the present invention, the substrate 1 may be a thinly oxidised and surface doped silicon substrate. With thinly oxidised is meant that on top of the substrate 1 a thin oxide layer 4 formed of oxidised material of the bulk substrate is present. In other words, in this case, the dielectric layer 4 is formed by a thin oxide layer which comprises an oxide of the material the bulk substrate is formed of. For example, if the bulk substrate 1 is formed of silicon, the thin oxide layer 4 on top of the bulk substrate 1 may be SiO₂. For embodiments featuring planar electrodes, the thickness of the insulation layer 4 may advantageously approach twice the characteristic length of measurement electrodes, defined as the sum of one electrode's width and spacing, in order to reduce the parasitic capacitance of the bulk mechanical support 2. In the case of micro-scaled electrodes, the surface oxide may be for example in the order of 30 nm only.

The electronic transducer or ISIT 10 according to embodiments of the present invention furthermore comprises, on top of the substrate 1, at least two access terminals forming a pair of sensing electrodes 5a and 5b, and a working area 6 (see Fig. 8a and 8b). The at least two insulated access terminals forming the sensing electrodes 5a, 5b may be formed of a conductive material, such as a metal, e.g. a non-noble metal of which aluminum is only one example, or they may be formed of a semiconductor material which may be, but is not necessarily, different from the sensing material, e.g. channel 3, in nature, i.e. in material, and/or in doping.

According to the present invention, the bottom surface of at least one of the sensing electrodes 5a, 5b is covered by a dielectric layer 4 of an insulating material. The dielectric layer 4 forms an electrical insulation between at least one of these sensing electrodes 5a, 5b and the layer of sensing material, e.g. channel 3, of the transducer 10. Hence, at least one of the sensing electrodes 5a, 5b is electrically insulated from the sensing material, e.g. channel 3, by the dielectric layer 4. Preferably, the bottom surface of both the sensing electrodes 5a, 5b is provided with a layer 4 of insulating material. The insulating material may be any suitable insulating material, such as e.g. an oxide or a nitride.
The working area 6 may include the top surface of the transducer 10, i.e. the top surface of the sensing electrodes 5a, 5b, and optionally may include other insulating or application specific layers 7 covering the transducers surface. Such non-conductive layer 7 may be formed by passivation, oxidation, nitridation or by depositing an insulating substance such as a paint of lacquer of similar insulating coating. If the metal of the electrodes 5a, 5b is aluminium, the insulating material may be alumina. In other words, according to embodiments of the present invention, the top surface of the sensing electrodes 5a, 5b may be covered by an insulating or application specific layer 7. For example, the top surface of the sensing electrodes 5a, 5b may be covered by a layer 7 which selectively binds specific target materials in order to increase the sensitivity of the transducer 10 for these specific target materials. According to other embodiments, the top surface of the transducer 10 may comprise conductive, e.g. metallic, or semiconductive particles or grains 9 (see Fig. 9), which function as labels, such as for example silver grains. Preferably, the conductive or semiconductive particles or grains 9 may be metallic particles. Therefore, in the further description, reference will be made to metallic particles 9 only. It has however to be understood that this is not limiting the invention in any way and that other conductive or semiconductive particles may also be used with the present invention. The surface coverage with such metallic particles may at least be between 0% and 30%, but may preferably be as high as possible, e.g. may reach 100%. These metallic particles or grains 9 have the function of labels and may also be provided for selectively binding specific target materials. The presence of metallic particles or grains 9 on the transducers surface leads to a decrease of capacitance cut-off frequency (see further).

A transducer device 10 according to embodiments of the present invention relies on the alignment between the Fermi level of a semiconductor sensing element, e.g. channel 3, for example silicon channel, and the workfunction of target metallic particles, e.g. silver grains, as in the ISFET case. According to the present invention, however, measurements are performed dynamically by impedance or dielectric relaxation time spectroscopy, instead of DC conduction or surface potential monitoring as known in the prior art. The absence of the requirement for ohmic contacts on sensing electrodes 5a, 5b is one advantage of the transducer or ISIT 10 according to embodiments of the present invention when compared to other, prior art compact devices such as ISFETs, since it allows smaller electrode dimensions. However, a main advantage of the transducer or ISIT 10 according to embodiments of the present invention is that their output impedance response to stimuli can be represented by a dielectric relaxation spectrum, which allows frequency multiplexing of parallel-connected ISITs 10 and furnishes additional information about the spatial distribution of stimuli on each ISIT 10. This is better than usual scalar measurements known in prior art, which are limited to an average of the signal over a complete working area.

An electronic transducer or ISIT 10 according to embodiments of the present invention may be integrated in a semiconductor substrate, for example silicon substrate, and uses impedance measurements for detecting modifications in a workfunction, redox potential ,Fermi-level or charge of target materials present at the surface of the electronic transducer 10, as well as electromagnetic, mechanical or temperature stimuli applied to the electronic transducer 10. The electronic transducers or ISITs 10 according to the present invention are easy to manufacture, allow low-cost impedance measurements with multiple parameters and open the way to dielectric relaxation constant spectroscopy within the scope of intelligent measurement systems on electronic chips.

According to embodiments of the present invention, the electronic transducer or ISIT 10 may comprise a first and a second electrically insulated access terminals which respectively form the sensing electrodes 5a and 5b, and a working area 6. According to one embodiment of the present invention, the first and second electrically insulated access terminals or sensing electrodes 5a, 5b and the working area 6 may be formed by two metal fingers, for example aluminium fingers, as illustrated in Fig. 8a and 8b. According to another, discrete embodiment of the present invention, the sensing electrodes 5a, 5b may be electrically connected to one terminal of respectively a discrete capacitor and a discrete sensing element defining the working area 6, both discrete elements otherwise being electrically connected in series.

According to other embodiments, the transducer or ISIT 10 may furthermore comprise an additional conductive electrode, or gate 5c, located at the top surface of the working area 6 (see Fig. 8b), which may be used for changing the impedance of the device 10 through charge or voltage modulation instead of labelling (see Fig. 8b). The presence of such a gate 5c, used as input terminal, may create a transducer device 10 where sensing electrodes 5a and 5b form the output terminals. The ISIT 10 may then be used to transform an electrical input vector such as a voltage or charge into an electrical output quantity, comprising a complex impedance with a real, resistive part and an imaginary, capacitive part.

According to other embodiments of the present invention, the electronic transducer or ISIT 10 may comprise a first, a second and a third electrically insulated access terminal, which respectively form sensing electrodes 5a, 5b and a back-gate electrode 5d. The third or back-gate electrode 5d (see Fig. 8a and 8b) may, as well as the sensing electrodes 5a, 5b, be made of a conductive material such as a metal, or of a semiconductor which may be, but is not necessarily, different from the sensing material, e.g. channel 3, in nature, i.e. in material, and/or in doping. The back-gate electrode 5d is electrically decoupled from the sensing material, e.g. channel 3, by means of a dielectric or resistive region such as, for example, an oxide or a semiconductor junction. The back-gate electrode 5d allows tuning of the working range of the transducer 10 and locating of a predominant electrical field region and may even lead to deactivation of the semiconductor channel 3, leading the transducer 10 to work as a classical electrode set on a passive support.

When, according to some embodiments of the present invention, the transducer or ISIT 10 comprises three electrodes, i.e. a pair of sensing electrodes 5a, 5b and a back-gate electrode 5d, AC measurements between one sensing electrode 5a or 5b and back-gate electrode 5d may, supplementary to inter-sensing-electrode sensing through the sensing material, also give information about the channel conductance and dielectric relaxation time distribution.

According to embodiments of the present invention, the sensing electrodes 5a, 5b and the working area 6 may be positioned at a first side of a substrate 1 and the back-gate electrode 5d may be positioned at a second side of the substrate 1, the first and second side being opposite to each other. If the entire substrate 1 is, for example, made of a semiconductor material, the associated back-gate access may itself substitute for the bulk mechanical support 2. Alternatively, if the substrate 1 is, for example, a Silicon-On-Insulator (SOI) membrane, e.g. formed over a cavity by locally removing the bulk mechanical support 2, a conductive layer deposited inside the cavity may form the back-gate electrode 5d.

A transducer 10 and method for detection and/or characterising of target materials according to the present invention are based on the intercalation of the dielectric layer 4 between one or both of the measurement electrodes (first and/or second access terminal or sensing electrodes 5a, 5b) and the sensing layer, e.g. channel 3. In case of the sensing layer being a channel 3, in a transducer 10 according to the present invention, the insulated sensing electrodes 5a, 5b therefore substitute drain and source DC contact regions of conventional transistors. From a fabrication point of view, this allows to possibly eliminate costly and delicate process steps that, for example, consist in opening contact holes between the electrodes 5a, 5b and the semiconductor through the dielectric layer 4.

With regard to conventional IDE, the main conceptual difference of the transducer or ISIT 10 according to the invention with respect to the prior art devices is that the sensing layer, e.g. channel 3, for example silicon channel, actively contributes to the measurement signal despite it is part of the substrate 1, which in IDE constitutes a noise source. Furthermore, back-gate voltage biasing can be exploited to change the transducer's working model by modifying the density and energy of charge carriers inside the sensing layer, e.g. channel 3. Back-gate voltage tuning can therefore concentrate current lines in the sensing layer, e.g. channel 3, by increasing the conduction of the material of the sensing layer, e.g. channel 3, or repel them toward an external reactive medium 8 to generate a quasi-IDE case, which is impossible to obtain with conventional CHEMFETs. The substrate voltage biasing may be applied on the backside of the substrate remote from the working area 6.

As already discussed, the transducers or ISITs 10 according to the present invention are therefore 2- to 4-terminal transducers comprising sensing electrodes 5a, 5b and optionally comprising a gate electrode 5c and/or a back-gate electrode 5d, which make the ISIT 10 according to embodiments of the present invention an intermediate device between transistors and IDE.

The principle of the detection method according to the present invention relies on an impedance measurement of the sensing layer, e.g. channel 3, conduction changes resulting from a stimulus. The impedance measurement is performed between the insulated sensing electrodes 5a, 5b. Target materials and events occurring at the working area 6 in the external medium 8, for example a solid (e.g. oxide), liquid (e.g. oil, water) or gaseous medium (e.g. air) present at the top surface of the sensing electrodes 5a, 5b and thus at the surface of the transducer 10, change the equivalent impedance of that medium 8 and influence the impedance of the sensing material, e.g. channel 3, of the transducer 10 indirectly by, for example, the field effect or optical interaction with incident light or directly by, for example, chemical reaction or mechanical constraints or any other stimulus relevant to the sensitive material, e.g. the material of the channel 3.

According to the method of the present invention, a first impedance of the sensing material, e.g. channel 3, is measured. Then, the external medium 8 comprising the target material to be detected and/or characterised is provided or introduced at the top surface of the transducer 10. The target material binds to the top surface of the transducer 10 or to a target specific layer 7 or metallic labels 9, e.g. silver grains, present at the surface of the transducer 10 and thereby changes the equivalent impedance of the external medium 8 and influences the impedance of the sensing material, e.g. channel 3, of the transducer 10. In a next step, a second impedance of the sensing material, e.g. channel 3, is measured, the second impedance being the impedance of the sensing material, e.g. channel 3, after the external medium 8 has been provided to the top surface of the transducer 10. In a subsequent step, the detection of presence and/or characterisation of the target material is done by determining the difference between the first impedance of the sensing material, e.g. channel 3, i.e. the impedance of the sensing material, e.g. channel 3, before provision of the target material, and the second impedance of the sensing material, e.g. channel 3, i.e. the impedance of the sensing material, e.g. channel 3, after provision of the target material.

Hereinafter, the principle of the method according to the present invention will be described making use of a semiconductor-based field-effect embodiment of an ISIT 10. The transducer 10 is based on detection of target materials through a complex impedance change between two electrically insulated electrodes 5a, 5b due to induction of charges in a semiconductor channel 3 nearby the electrodes 5a, 5b but insulated therefrom, which semiconductor channel 3 forms the sensing element of the transducer 10. Charge induction is generated because of the presence of target materials that, according to the present example, have a specific workfunction, and which are present on top of the insulated channel 3. Optionally, a third, back-gate electrode 5d may be present.

Fig. 9 illustrates a 2D cross-section perpendicularly to the first and second access terminals forming respectively the sensing electrodes 5a and 5b (see left part of Fig. 9) of a transducer or ISIT 10 according to an embodiment of the present invention and the corresponding electrical model (see right part of Fig. 9). The AC coupling introduced by the electrode/sensing material, e.g. channel 3, insulation results in a complex impedance dipole formed of a surface capacitance C in series with the sensing material's, e.g. channel's, resistance R. Such a lateral dipole is characterized by a dielectric relaxation time equal to the RC product. Since the surface capacitance C is designed and fixed at fabrication of the transducer 10, the RC time constant evolves as a function of external stimuli, as these stimuli modulate or change the sensing material's, e.g. channel's, resistance. Once extended in the 3^{rd} dimension along the electrodes 5a, 5b, such structure can be modelled as a distributed array of dipoles, whose relaxation time values spread as much as the heterogeneity of the stimuli increases. The density or intensity of a stimulus at one point of the working area 6 creates a unique, associated, relaxation time, so that a sum of different stimuli having various densities or intensities over the working area 6 creates a response spectrum containing a lot of different relaxation times.

Fig. 10 (left part) shows a SEM photograph of a top view of a device according to an embodiment of the present invention, on which microlabels 9 are bound. The right part of Fig. 10 illustrates a distributed model corresponding with the transducer 10 illustrated in the left part of Fig. 10. Measurements involving dielectric relaxation time spectroscopy then provide a way to extract the distributed sheet resistance of the measurement material, e.g. channel [W. Strunz et al., Electrochimica acta in press (2006)]. This constitutes an advantageous innovation of the ISIT concept, which produces a relaxation spectrum that depends on the heterogeneity of the stimuli all over the work area 6 (Fig. 11). The right-hand side of Fig. 11 illustrates prior art calculation results of the dielectric relaxation spectrum corresponding to a lumped elements model, as shown in the left hand side of Fig. 11. Each positive peak of the computed curve corresponds to a dielectric couple made of a resistance Rx in series with a capacitance Cx. Applying such algorithm to ISIT measurements will permit to extract dielectric relaxation constants associated to its electrical model, shown in right side of Fig. 10, for instance. In such example, the computed dielectric spectrum is therefore an image of the labels size distribution visible in the left hand side of Fig. 10.

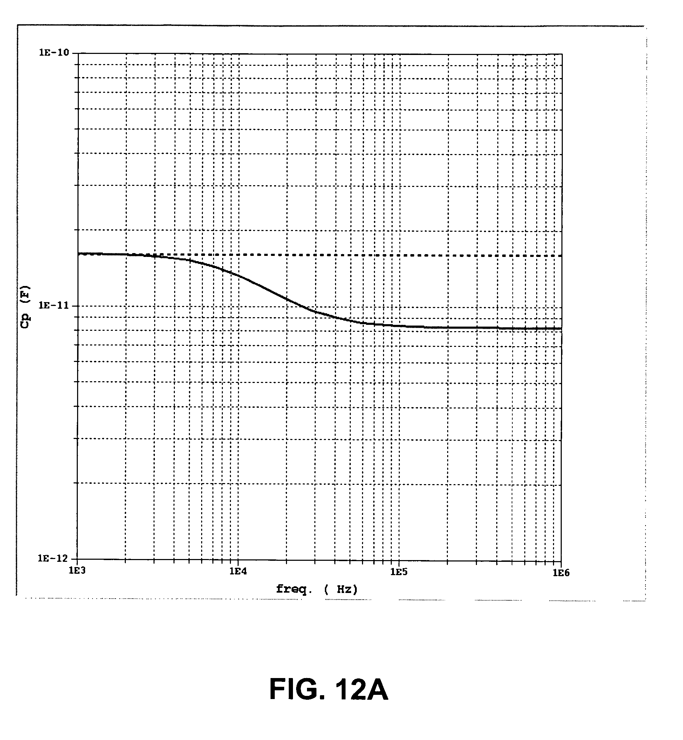

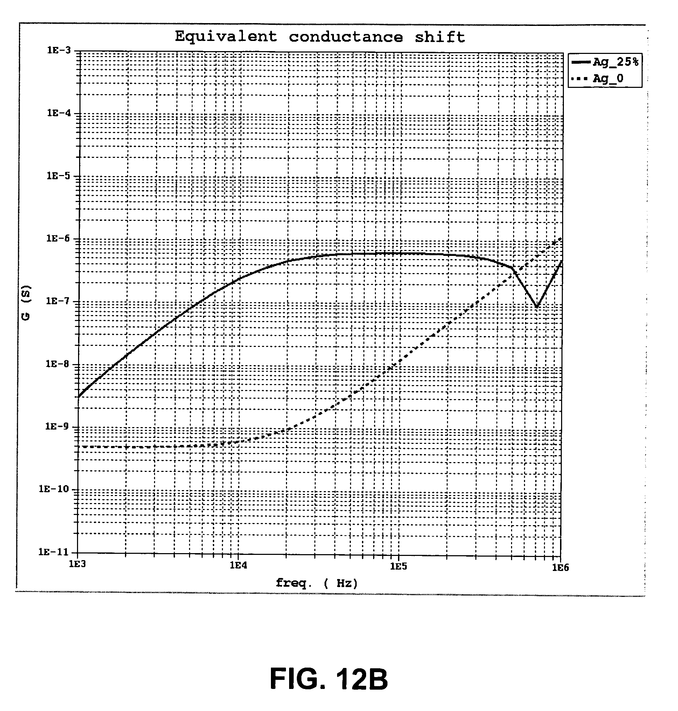

To demonstrate field-effect sensing with semiconductor-based ISITs, according to an embodiment of the present invention, hereinafter 2D finite-element simulations are performed using a transducer 10 comprising two aluminium fingers forming the sensing electrodes 5a, 5b and a working area 6. These simulations indicate the qualitative influence of metallic micro-labels or grains 9, in the example given silver grains, (see left part of Fig. 9) on real and imaginary parts of the interdigit impedance. The graph of Fig. 12A shows the equivalent parallel capacitance Cp as a function of frequency for a blank transducer surface, i.e. for a transducer surface without the presence of silver grains 9, (dashed line), and for a transducer surface comprising silver grains 9 which cover about 25% of the surface (full line). It can be seen from this graph that the equivalent parallel capacitance Cp curve shifts to lower frequencies with the presence of such labels or grains 9, which means that the apparent capacitance cut-off frequency decreases. In the same time, the parallel equivalent conductance G increases with increasing density of the grains 9 and thus with increasing surface coverage of the grains 9. This is illustrated in the graph of Fig. 12B, which illustrates the parallel equivalent conductance G as a function of the frequency for a blank transducer surface, i.e. for a transducer surface without the presence of silver grains 9 (dashed line), and for a transducer surface comprising silver grains 9 which cover about 25% of the surface (full line).

From the above it can be concluded that transducers 10 according to embodiments of the present invention feature at least two detection degrees, i.e. conductance and capacitance, and even more when considering their frequency dependence. This leads to integrated smart sensing by on-chip impedance spectroscopy, which is a much more powerful method than the pure resistive or capacitive traditional approaches known in the art.

It has to be noted that also other metallic labels or grains 9 than the silver grains in the above example can be used for the above-described purpose, such as, for example, gold particles, iron particles, lead particles,as well as semiconductor crystals or quantum dots formed of e.g. cadmium, selenium or zinc, etc.

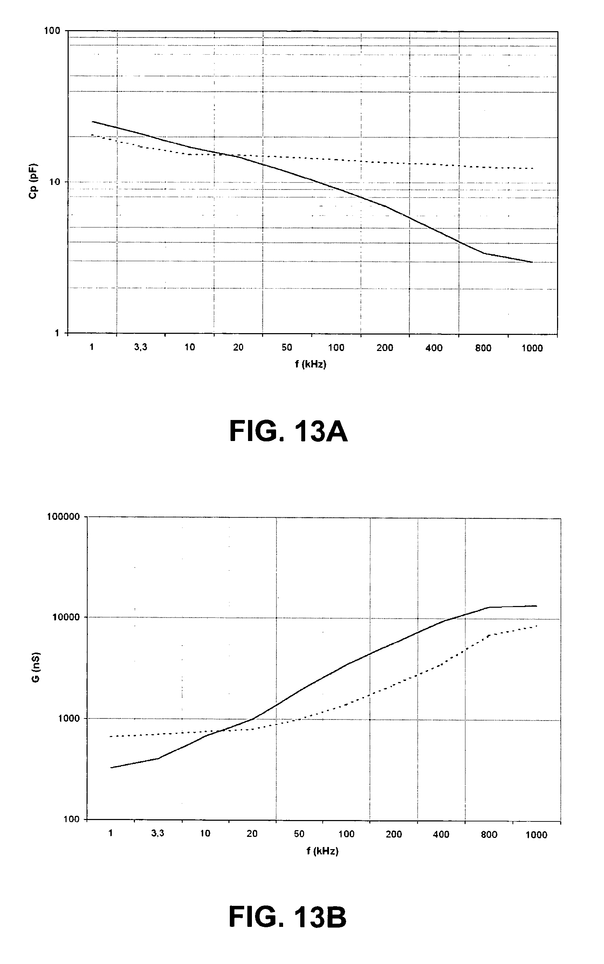

The simulated tendency described above has been confirmed by experiments. The results of these experiments are shown in Fig. 13A and 13B. Positive measurements were obtained on six different transducers 10. The measurement comprised detecting 1 nM of 534-mer single-stranded DNA targets through a global admittance change of a factor 3 corresponding to 7 pF maximal capacitance decrease (- 70 % regarding base value, i.e. value without target being present).

According to the present invention, the transducers or ISITs 10 can also be formed on silicon-on-insulator or SOI substrates in order to reduce the influence of the semiconductor substrate 1, especially when this substrate 1 is formed of silicon, ultimately leading to SOI membranes. In these cases, back-gate voltage biasing may be done by means of metal deposition on the membrane's back-side directly.

It has to be noted that, notwithstanding in the above discussion, the working principle of the transducer or ISIT 10 according to the present invention has been demonstrated on the basis of the stimulus being a field-effect, the present invention also applies for any other stimulus applied such as, for example, a chemical reaction in or with the external medium 8, physical or mechanical constraint applications such as e.g. temperature-enhanced chemical reaction of ethanol with SnO₂, stress of a semiconductor-based microcantilever, etc., light illumination, ions bombardement, etc. Furthermore, any material featuring a variable conductance when exposed to such stimuli may be used to form the sensing material, e.g. channel 3 of the transducer 10.

The present invention can be applied to a lot of new applications by considering spectroscopic multiplexing as follows: given a sensing material's, e.g. channel's, resistance range {Rₘᵢₙ ; Rₘₐₓ} and a surface capacitance C₀, the time constants interval of an ISIT is {Rₘᵢₙ.C₀ ; Rₘₐₓ.C₀}. When connecting several transducers or ISITs 10 in parallel with different surface capacitances {C₁ C₂ .. Cₙ} such that Rₘₐₓ.Cₖ < Rₘᵢₙ.Cₖ₊₁, one single measurement of the complete array could retrieve the information stored in each ISIT 10 by observing, in the full dielectric spectrum of the whole array, the only range [Rₘᵢₙ.Cᵢ; Rₘₐₓ.Cᵢ] of relaxation constants corresponding to the ISIT number i.

The transducer or ISIT 10 and the method for detecting and/or characterising target materials according to the present invention has some advantages with respect to prior art devices.

Classical electrodes patterned on an insulated support and in contact with the external medium to be tested only address the extrinsic measurement of external medium properties. The transducer or ISIT 10 according to the present invention offers an additional possibility to measure the interaction between an external medium 8 and the sensing material, e.g. channel 3, for example silicon channel, of the transducer 10 and preserves the external medium 8 from current flow alterations, as measurement current will flow through the sensing material and not through the external medium 8.

Contrary to CHEMFET transducers, e.g. ISFET transducers, the transducer or ISIT 10 according to the present invention needs no ohmic drain and source access and facilitates on-chip AC impedance spectroscopy instead of DC monitoring as applied to CHEMFET transducers. Hence, the transducer or ISIT 10 according to the present invention provides both spatial and temporal information related to distributed conduction states and subsequent dielectric relaxation constants throughout the sensing material, e.g. channel 3, for example silicon channel, of the transducer 10. Regarding such on-chip dielectric spectroscopy, the dielectric layer 4 plays a crucial role as a reference capacitive element.

It is a further advantage that the transducer or ISIT 10 according to the present invention is easy to manufacture and can be manufactured at low-cost. Furthermore, the transducer or ISIT 10 according to the present invention shows a good sensitivity and a low detection limit which may be smaller than, for example, 1 nM in case of 30bp DNA targets.

Furthermore, the method according to the present invention is less destructive with respect to prior art methods because, according to the method of the invention, the major part of the current is centralised in the sensing element, e.g. channel 3. In some of the prior art documents, for example, where capacitive measurements are performed as described in EP 1 376 111, the current circles in the external environment, where it is able to degrade the medium the target particles are present in or the surface of the sensor by stimulating, for example, chemical attacks (parasitic faraday current) and thereby increasing the noise of the measurement.

The method and device according to the present invention can be used with all materials comprising a work function, or in other words, most of the materials which are not pure insulators.

Furthermore, the technology proposed by this invention is compatible with other measurement methods known in the art, such as impedance spectroscopy.

It is to be understood that although preferred embodiments, specific constructions and configurations, as well as materials, have been discussed herein for devices according to the present invention, various changes or modifications in form and detail may be made without departing from the scope and spirit of this invention.

## Claims

1. A solid-state electronic transducer (10) for testing an external medium, the electronic transducer (10) comprising:
- a sensing element (3) made of sensing material featuring a variable conductance when exposed to a stimulus from the external medium, and
- a first and a second electrode (5a, 5b) spaced apart on or in a sensing material surface of a substrate, the sensing element being provided in or on the substrate and being located between the first and the second electrodes (5a, 5b), the first and the second electrodes (5a, 5b) forming a pair of sensing electrodes for sensing a change in conductance of the sensing element (3) in a direction substantially parallel to the sensing material surface,
wherein at least one of the first and second electrodes (5a, 5b) is electrically insulated from the sensing element (3) by a dielectric layer (4), so as to be capacitively coupled to the sensing element.

2. A solid-state electronic transducer (10) according to claim 1, wherein the first and second electrodes (5a, 5b) are such that a measurement signal applied to these electrodes does not change the conductance of the sensing element (3).

3. A solid-state electronic transducer (10) according to claim 1 or 2, the transducer (10) furthermore comprising a target specific insulating layer (7) covering at least the sensing element (3) and/or the sensing electrodes (5a,5b) and insulating it from the external medium, the insulating layer (7) being adapted to contact with the external medium.

4. A solid-state electronic transducer (10) according to any of the previous claims, furthermore comprising a third access terminal forming a back-contact or back-gate electrode (5d) at a side of the substrate different from the side where the sensing element (3) is provided.

5. A solid-state electronic transducer (10) according to any of the previous claims, wherein the sensing electrodes (5a,5b) are both electrically insulated from the sensing element (3) by the dielectric layer (4).

6. A solid-state electronic transducer (10) according to any of the previous claims, wherein the sensing electrodes (5a,5b) are formed of a conductive or semiconducting material.

7. A solid-state electronic transducer (10) according to claim 6, wherein the sensing electrodes (5a,5b) are formed of aluminium fingers.

8. A solid-state electronic transducer (10) according to any of the previous claims, wherein substrate is an oxidised doped semiconductor substrate.

9. A solid-state electronic transducer (10) according to any of the previous claims, wherein the sensing material is a semiconductor material.

10. A solid-state electronic transducer (10) according to claim 9, wherein the sensing material is a doped semiconductor material.

11. A solid-state electronic transducer (10) according to any of claims 8 to 10, wherein the sensing material forms a channel (3) in the substrate (1).

12. A solid-state electronic transducer (10) according to any of the previous claims, wherein the dielectric layer (4) comprises one insulator material, a combination of insulator materials, one semiconductor material having dielectric properties, a combination of semiconductor materials having dielectric properties, or a combination of at least one insulator material and at least one semiconductor material having dielectric properties.

13. A solid-state electronic transducer (10) according to claim 12, wherein the insulator material is one of an oxide, a nitride or a polymer.

14. A solid-state electronic transducer (10) according to any of the previous claims, the transducer (10) having a top surface, wherein the top surface of the transducer (10) is provided with conductive or semiconductive particles (9).

15. A solid-state electronic transducer (10) according to claim 14, wherein the conductive or semiconductive particles (9) are metallic particles.

16. A method for sensing, characterising and/or detecting the presence of target materials or physico-chemical stimuli in an external medium (8), the method comprising:
- measuring a first impedance of a sensing element (3) of a solid-state electronic transducer (10), the electronic transducer (10) furthermore comprising a first and second access terminal forming sensing electrodes (5a,5b) for sensing conductance of the sensing element (3), at least one of the sensing electrodes (5a,5b) being electrically insulated from the sensing element (3) by a dielectric layer (4) so as to be capacitively coupled to the sensing element (3),
- providing the medium (8) comprising the target material to a surface of the transducer (10),
- measuring a second impedance of the sensing element (3) after providing the medium to the electronic transducer (10), and
- from the difference between the first and second impedances of the sensing element (3) determining the presence of and/or characterising the target material.

17. A method according to claim 16, wherein the target material is a conductive compound, an insulating material, a semi-conducting material, a biochemical species, an ionised atom or molecule, a charged particle appearing in solid, liquid or gaseous media.

18. A method according to claim 16, wherein the physico-chemical stimulus is any of light, temperature, pressure, flow, mass, magnetic stimulus, mechanical stimulus, or electrical stimulus.
